# EUROPÄISCHE PATENTSCHRIFT

(11) **EP 1 958 656 B1**
(45) Veröffentlichungstag und Bekanntmachung des Hinweises auf die Patenterteilung: **23.11.2016**
(21) Anmeldenummer: 08002133.0
(22) Anmeldetag: 05.02.2008
(51) Int. Cl.: A61M 25/00, A61M 25/01

(54) **Verfahren zum Benetzen eines hydrophilen Urinal-Katheters sowie zugehöriges Kathetersystem**
Method for wetting a hydrophilic urine catheter and corresponding catheter system
Procédé de mouillage d'un cathéter urinaire hydrophile et système de cathéter correspondant

(30) Priorität: 13.02.2007 DE 102007006905
(43) Veröffentlichungstag der Anmeldung: 20.08.2008
(73) Patentinhaber: Medical Service GmbH, 75378 Bad Liebenzell (DE)
(72) Erfinder:
(74) Vertreter: Grünecker Patent- und Rechtsanwälte PartG mbB

(56) Entgegenhaltungen:
- WO-A-99/30761
- WO-A-03/092779
- US-A- 3 967 728
- US-A1- 2001 001 443
- US-A1- 2005 109 648
- US-A1- 2006 196 783

## Beschreibung

Die Erfindung betrifft ein Verfahren zur Benetzung eines hydrophilen Urinal-Katheters innerhalb einer geschlossenen Verpackung des Katheters. Die Erfindung betrifft weiterhin ein Kathetersystem, umfassend
- einen Urinal-Katheter mit einer hydrophilen Beschichtung und
- eine Verpackung, in der der Katheter angeordnet ist, wobei die Verpackung aufweist
- einen länglichen Benetzungsbereich, der zumindest einen zu benetzenden Teil des Katheters enthält, und
- einen Vorratsbereich mit einem Depot für eine Benetzungsflüssigkeit, wobei das Depot geöffnet werden kann, so dass die Benetzungsflüssigkeit vom Vorratsbereich durch einen Übergangsbereich in den Benetzungsbereich fließen kann.

Ein solches Verfahren und ein Kathetersystem sind beispielsweise bekannt geworden durch WO 2006/092150 A1 und die hierzu korrespondierende US 2006/196783 A1.

Urinal-Katheter werden dazu eingesetzt, bei einem Patienten die Harnblase zu entleeren. Der Katheter wird dazu in die Harnröhre bis in die Harnblase des Patienten eingeschoben. Durch den Katheter, der im Wesentlichen als hohler Schlauch ausgebildet ist, kann der Urin abfließen.

Um das Einführen des Katheters für den Patienten möglichst schmerzfrei zu gestalten, ist es bekannt, die Oberfläche des Katheters, zumindest soweit er in die Harnröhre eingeschoben werden soll, besser gleitfähig zu machen. Eine sehr gute Gleitfähigkeit kann durch so genannte hydrophile Katheter erreicht werden. Diese Katheter weisen auf ihrer Außenseite eine hydrophile Beschichtung auf, die durch eine wässrige Benetzungsflüssigkeit (etwa eine 0,9%ige wässrige NaCl-Lösung) aktiviert und damit schlüpfrig gemacht werden kann. Der hydrophile Katheter wird dabei kurz vor dem Einführen in die Harnröhre für ca. 30 Sekunden mit der Benetzungsflüssigkeit in Kontakt gebracht.

Sowohl der hydrophile Katheter selbst als auch die Benetzungsflüssigkeit müssen steril sein, um eine Infektion des Patienten, bei dem der Katheter eingeführt wird, auszuschließen. Viele Patienten führen ihre Katheterisierung selbst durch, insbesondere auch unterwegs. Für diese Anwendung sind Kathetersysteme bekannt geworden, bei denen eine Benetzung des Katheters innerhalb einer geschlossenen, sterilen Verpackung des Katheters ohne direkten Kontakt mit den Fingern durchgeführt werden kann. Innerhalb der Verpackung ist dazu ein Vorrat an steriler Benetzungsflüssigkeit vorhanden, der auf den Katheter entleert werden kann.

Die WO 2006/092150 A1 beinhaltet ein Kathetersystem, bei dem die Verpackung sowohl einen hydrophilen Katheter als auch ein Depot für ein Aktivierungsmittel der hydrophilen Schicht des Katheters umfasst. Das Depot wird durch den Benutzer vor bzw. bei Öffnung der Verpackung geöffnet, wodurch der Katheter in seiner Verpackung mit dem Aktivierungsmittel in Kontakt gelangt.

Aus der EP 0 959 930 B1 ist ein Kathetersystem bekannt geworden, bei welchem die Verpackung eine längliche Tasche aufweist, in welche ein hydrophiler Katheter mit seinem Einführende voraus mit einem vorderen Teil eingelegt ist. In einem als Urinbeutel ausgebildeten Teil der Verpackung, in welchen die längliche Tasche trichterartig mündet, ist ein Flüssigkeitsdepot vorgesehen. Zum Aktivieren des Katheters wird das Kathetersystem mit der länglichen Tasche senkrecht nach unten gerichtet gehalten und das Depot geöffnet. Die Benetzungsflüssigkeit fließt in die Tasche und füllt diese auf, wobei der in der Tasche befindliche Teil des Katheters benetzt wird. Nach Ablauf einer Wartezeit wird der Katheter verwendet.

Eine weitere Benetzungsvorrichtung für hydrophile Harnkatheter wird in der DE 29724914 U1 genannt. Der hydrophile Harnkatheter ist dabei in einem Behältnis angeordnet, in den ein Benetzungsfluidbehälter integriert ist. Nach Öffnung des Fluidbehälters wird die einführbare Länge des Harnkatheters mit dem Fluid benetzt. Zudem dient die Verpackung zugleich als Auffangbehälter für Urin.

Die DE 10213411 B4 nennt eine Verpackung für Katheter, die ein Gleitmittel enthält, das medizinische Wirkstoffe enthält. Bei Herausschieben des Katheters aus der Verpackung passiert der Katheter das Gleitmitteldepot und wird dabei mit Gleitmittel benetzt.

Die EP 0677299 B1 beinhaltet eine Verpackung für einen Katheter, wobei der Katheter bei Entnahme aus der Verpackung mit Gleitmittel bzw. Benetzungsmittel für eine hydrophile Schicht benetzt wird. Das Gleitmitte bzw. Benetzungsmittel kann sich dabei bereits in der Verpackung befinden oder wird nach der Öffnung der Verpackung durch den Nutzer hineingegeben.

In der EP 0252918 B1 wird eine Befeuchtungs- und Lagervorrichtung für einen Katheter beschrieben, die einen Bereich umfasst, in dem der Katheter befeuchtet werden kann und auch einen gesonderten weiteren Bereich umfasst, in dem Katheter aufbewahrt werden kann.

Nachteilig bei diesem Stand der Technik ist die relativ große Menge an Benetzungsflüssigkeit, die zum Aktivieren des Katheters benötigt wird. Die längliche Tasche muss soweit aufgefüllt werden, wie die zum Einführen in die Harnröhre benötigte Katheterlänge reicht. Die große Menge der sterilen Benetzungsflüssigkeit erhöht die Herstellungskosten des Kathetersystems. 10 Weiterhin erhöht die große Menge an Benetzungsflüssigkeit das Gewicht des Kathetersystems, wodurch unter anderem das Mitführen des Kathetersystems für den Patienten unterwegs beschwerlicher wird.

### Aufgabe der Erfindung

Es ist daher die Aufgabe der vorliegenden Erfindung, ein Verfahren zur Benetzung eines hydrophilen Katheters innerhalb einer geschlossenen Verpackung des Katheters vorzustellen, welches mit einer geringeren Menge an Benetzungsflüssigkeit auskommen kann. Ebenso soll ein Kathetersystem vorgestellt werden, bei welchem die notwendige Menge an mitgeführter Benetzungsflüssigkeit reduziert ist, ohne die Benetzungsfähigkeit zu beeinträchtigen.

### Kurze Beschreibung der Erfindung

Diese Aufgabe wird in Hinblick auf das Benetzungsverfahren gelöst durch ein Verfahren zur Benetzung eines hydrophilen Urinal-Katheters innerhalb einer geschlossenen Verpackung des Katheters, wobei die Verpackung aufweist
- einen länglichen Benetzungsbereich, der zumindest einen zu benetzenden Teil des hydrophilen Urinal-Katheters enthält, und
- einen Vorratsbereich mit einem Depot für eine Benetzungsflüssigkeit,
wobei das Depot geöffnet werden kann, so dass die Benetzungsflüssigkeit vom Vorratsbereich durch einen Übergangsbereich in den Benetzungsbereich fließen kann, wobei das Verfahren folgende Schritte umfasst:
a) das Depot wird geöffnet, insbesondere durch Ausübung eines äußeren Drucks auf das Depot durch die Verpackung;
b) die Benetzungsflüssigkeit fließt aus dem Vorratsbereich in ein freies Volumen des länglichen Benetzungsbereich und füllt dieses zu maximal 2/3 mit Benetzungsflüssigkeit, insbesondere fließt die Benetzungsflüssigkeit durch Schwerkraft zu einem Ende des länglichen Benetzungsbereichs, das vom Übergangsbereich abgewandt ist, wobei das freie Volumen Vfrei das Volumen Vbb des Benetzungsbereichs, abzüglich des Volumens Vkat des im Benetzungsbereich angeordneten Teils des Katheters ist;
c) der Übergangsbereich wird abgedichtet, so dass ein Rückfluss von Benetzungsflüssigkeit aus dem Benetzungsbereich in den Vorratsbereich zumindest weitgehend blockiert ist;
d) die Verpackung wird geschwenkt, insbesondere so dass die Benetzungsflüssigkeit durch Schwerkraft zu einem Ende des länglichen Benetzungsbereichs fließt, welches dem Übergangsbereich nahegelegen ist.

Im Rahmen der Erfindung wird ausgenutzt, dass es für die Aktivierung eines hydrophilen Katheters nicht notwendig ist, den zu aktivierenden Teil des Katheters während der Aktivierungszeit *vollständig und andauemd* in der Benetzungsflüssigkeit zu halten. Vielmehr ist es ausreichend, wenn alle Abschnitte des zu aktivierende Teils des Katheters einmal intensiv mit Benetzungsflüssigkeit getränkt werden (etwa zu Beginn der Aktivierungszeit) und der Katheter erst nach Ablauf der Aktivierungszeit verwendet wird. Insbesondere ist es erfindungsgemäß möglich, verschiedene Abschnitte des zu aktivierenden Teils des Katheters *nacheinander mit derselben Benetzungstlüssigkeit zu* tränken. Dadurch kann Benetzungsflüssigkeit eingespart werden.

Im Rahmen der Erfindung ist dafür zunächst vorgesehen, in den Benetzungsbereich der Verpackung, in dem der zu benetzende Teil des Katheters (oder auch der ganze Katheter) angeordnet ist, die Benetzungsflüssigkeit aus dem Depot einzuleiten. Dadurch wird ein erster, bei der gegenwärtigen Orientierung der Verpackung unterer Teil des Benetzungsbereichs überflutet, und ein erster Abschnitt des Katheters, der im überfluteten Teil angeordnet ist, wird benetzt. Bevorzugt macht dieser erste, untere Teil des Benetzungsbereichs etwa die Hälfte des freien (d.h. nicht schon durch den Katheter eingenommenen Teil des) Volumens des Benetzungsbereichs aus.

Soweit bei diesem Einleiten Benetzungsflüssigkeit nur einmalig am Katheter vorbeirinnt, ist dies für eine zuverlässige Aktivierung der hydrophilen Beschichtung noch nicht ausreichend. Daher muss nun der übrige zu benetzende Teil des Katheters intensiv getränkt werden.

Um die Benetzungsflüssigkeit mit anderen Katheterteilen in Kontakt zu bringen, d.h. um die Benetzungsflüssigkeit in andere Teile des Benetzungsbereichs fließen zu lassen, könnte die Verpackung verschwenkt werden. Allerdings besteht dabei die Gefahr, dass die Benetzungsflüssigkeit zurück in den Vorratsbereich der Verpackung fließt und nichts zur Benetzung des Katheters beitragen kann. Insbesondere derjenige Teil des Benetzungsbereichs, der nahe beim Übergangsbereich zum Vorratsbereich liegt, könnte nicht für ausreichende Zeit überflutet bzw. umspült werden.

Daher sieht die Erfindung vor, spätestens nach dem Einlassen der Benetzungsflüssigkeit in den Benetzungsbereich den Übergangsbereich gegenüber einem Rückfluss von Benetzungsflüssigkeit in den Vorratsbereich abzudichten. Im einfachsten Fall kann dazu der Verwender des Kathetersystems manuell eine Abdichtung vornehmen (etwa durch Abklemmen), oder aber die Abdichtung erfolgt aufgrund der Bauweise des Kathetersystems automatisch (etwa mittels eines Ventils, insbesondere Flatterventils).

Nach dem Abdichten des Übergangsbereichs kann die Verpackung problemlos verschwenkt werden, um die Benetzungsflüssigkeit zu einem anderen Teil des Benetzungsbereichs fließen zu lassen. Beispielsweise wird nun die Verpackung gedreht, so dass der bisher untere Teil des länglichen Benetzungsbereichs nach oben gerichtet ist, und der bisher obere Teil des Benetzungsbereichs nach unten gerichtet ist. In dem bevorzugten Fall, dass die Menge an Benetzungsflüssigkeit etwa der Hälfte des freien Volumens des Benetzungsbereichs entspricht, wird dadurch der übrige, bisher noch nicht überflutete Teil des Benetzungsbereichs überflutet; dadurch ist eine ausreichende Aktivierung eines jeglichen zu benetzenden Teils des Katheters innerhalb des Benetzungsbereichs sichergestellt.

Das für eine Aktivierung eines Abschnitts der hydrophilen Beschichtung eines Katheters ausreichende intensive Tränken mit Benetzungsflüssigkeit ist insbesondere gewährleistet, wenn der Abschnitt einmalig kurzzeitig (schon 2-3 Sekunden reichen bei gängigen Beschichtungen) überflutet war.

Ein intensives Tränken kann im Rahmen der Erfindung aber auch durch am Abschnitt vorbeifließende Benetzungsflüssigkeit erreicht werden, insbesondere wenn das Vorbeifließen langsam und wiederholt eingerichtet wird. Da nach der Abdichtung des Übergangsbereichs die Benetzungsflüssigkeit nur noch im Benetzungsbereich fließen kann, kann jegliche Verschwenkung der Verpackung ohne Flüssigkeitsverlust erfolgen. Insbesondere kann die Verpackung mit dem länglichen Benetzungsbereich näherungsweise horizontal gehalten werden und mit den beiden Enden des Benetzungsbereichs langsam abwechselnd leicht nach unten gekippt werden, beispielsweise mit einer Frequenz von ca. einer Verkippung pro Sekunde und einem Verkippungswinkel aus der Horizontalen von ca. 5-15°. Dabei fließt die Benetzungsflüssigkeit zwischen den beiden Enden des länglichen Benetzungsbereichs hin und her. Insbesondere ein in der Mitte des Benetzungsbereichs angeordneter Katheterteil kann durch diese Technik für eine Aktivierung einer hydrophilen Beschichtung ausreichend umspült werden.

Im Rahmen der Erfindung ist es nicht notwendig, die Abdichtung des Obergangsbereichs völlig flüssigkeitsdicht vorzunehmen. Ein Rückfluss, der für die Gewährleistung einer ausreichenden Aktivierung des Katheters unbeachtlich ist, kann außer Betracht bleiben. In der Regel ist ein Flüssigkeitsverlust von bis zu ca. 20% der Benetzungsflüssigkeit über einen typischen Aktivierungszeitraum von 30 Sekunden unbeachtlich.

Es versteht sich, dass im Rahmen der Erfindung der Benetzungsbereich während der Aktivierung der hydrophilen Beschichtung insgesamt allenfalls unbeachtliche Undichtigkeiten aufweisen darf. Bevorzugt weist der Benetzungsbereich beim Aktivieren der hydrophilen Beschichtung daher außer dem Übergangsbereich keine weiteren Öffnungen auf. Der Benetzungsbereich schließt erfindungsgemäß ein möglichst geringes Volumen um den Katheter, so weit dieser im Benetzungsbereich angeordnet ist ein, so dass die Benetzungsflüssigkeit effizient genutzt werden kann.

In Schritt b) wird das freie Volumen des Benetzungsbereichs, das ist das Volumen Vbb des Benetzungsbereichs abzüglich des Volumens Vkat des Teils des Katheters, welcher im Benetzungsbereich angeordnet ist, maximal zu 2/3 mit Benetzungsflüssigkeit gefüllt. Dadurch wird eine Ersparnis an Benetzungsflüssigkeit realisiert. Für ein einfaches, zweistufiges Benetzen ist ein Flüssigkeitsvolumen Vfl=1/2*Vfrei ausreichend; durch geringfügig höhere Mengen an Flüssigkeit können verbleibende Undichtigkeiten des Benetzungsbereichs ausgeglichen werden.

In den Rahmen der vorliegenden Erfindung fällt auch ein Kathetersystem, umfassend
- einen Urinal-Katheter mit einer hydrophilen Beschichtung und
- eine Verpackung, in der der Katheter angeordnet ist, wobei die Verpackung aufweist
- einen länglichen Benetzungsbereich, der zumindest einen zu benetzenden Teil des Katheters enthält, und
- einen Vorratsbereich mit einem Depot für eine Benetzungsflüssigkeit, wobei das Depot geöffnet werden kann, so dass die Benetzungsflüssigkeit vom Vorratsbereich durch einen Übergangsbereich in den Benetzungsbereich fließen kann, und der Übergangsbereich abdichtbar ausgebildet ist, so dass nach dem Öffnen des Depots ein Rückfluss von Benetzungsflüssigkeit aus dem Benetzungsbereich in den Vorratsbereich weitgehend verhindert werden kann, und für das Volumen Vfl der Benetzungsflüssigkeit des Depots, das Volumen Vbb des Benetzungsbereichs und das Volumen Vkat des Teils des Katheters, welcher im Benetzungsbereich angeordnet ist, gilt:
   Vfl ≤ 2/3*(Vbb-Vkath), und bevorzugt Vfl ≤ 1/2*(Vbb-Vkat). Ein solches erfindungsgemäßes Kathetersystem kann eine Benetzung des gesamten zu aktivierenden Teils des Katheters sicherstellen, ohne dass der gesamte zu aktivierende Teil des Katheters nach der Entleerung des Depots überflutet ist. Dadurch kann Benetzungsflüssigkeit eingespart werden.

### Bevorzugte Varianten der Erfindung

Bei einer bevorzugten Variante des erfindungsgemäßen Verfahrens ist vorgesehen, dass nach Schritt d) wenigstens 15 Sekunden, bevorzugt wenigstens 30 Sekunden, vergehen, bevor der Katheter ausgepackt und in die Harnröhre eines Patienten eingeführt wird. Diese Zeit ist in der Regel zum Aktivieren der hydrophilen Beschichtung (Quellung und chemische Prozesse) ausreichend, so dass eine gute Schlüpfrigkeit der hydrophilen Beschichtung erreicht wird.

Bevorzugt ist auch eine Variante, bei der nach Schritt d) ein weiterer Schritt e) erfolgt, mit: e) Die Verpackung wird hin- und hergeschwenkt, so dass die Benetzungsflüssigkeit im länglichen Benetzungsbereich zwischen dessen Enden hin- und herfließt. Bei dieser Variante reicht eine besonders kleine Menge an Benetzungsflüssigkeit aus. Diese Variante wird angewandt, um bei einem Volumen Vfl an Benetzungsflüssigkeit, das kleiner ist als die Hälfte des freien Volumens Vfrei des Benetzungsbereichs (mit Vfrei=Vbb-Vkat) eine Aktivierung eines Beschichtungsabschnitts des Katheters in der Mitte des Benetzungsbereichs zu gewährleisten. Dieser Benetzungsabschnitt wird in Schritt e) mehrfach von Benetzungsflüssigkeit umspült (die Endabschnitte des Benetzungsbereichs und damit etwaige dortige Katheterabschnitte können ohnehin überflutet werden).

Bevorzugt ist auch eine Verfahrensvariante, bei der in Schritt c) die Verpackung zum Abdichten am Übergangsbereich umgeknickt wird. Dadurch wird das Abdichten besonders einfach, und kann insbesondere ohne von der Verpackung separate Hilfsmittel erfolgen.

Bevorzugt wird durch den oben beschriebenen Schritt b) das Flüssigkeitsvolumen Vfl kleiner als %*Vfrei (also maximal zu ½) gewählt. In der Regel sollte aber Vfl wenigstens 20% von Vfrei betragen.

Eine bevorzugte Ausführungsform des erfindungsgemäßen Kathetersystems sieht vor, dass die Verpackung im Übergangsbereich zwischen Vorratsbereich und Benetzungsbereich abknickbar ausgebildet ist, insbesondere entlang einer markierten Biegelinie. Bei dieser Ausführungsform ist die Verpackung ausreichend flexibel, um die Verpackung leckfrei umknicken zu können, wobei durch das Umknicken eine weitgehende Blockierung (Abdichtung) des Übergangsbereichs erzielt wird. Die Biegelinie ist in der Regel nicht nur markiert (etwa mittels eines Aufdrucks). In der Regel ist die Biegelinie durch die Ausformung der Verpackung kenntlich, und nur bei Abknicken an der Biegelinie tritt eine optimale Dichtwirkung ein.

Bei einer bevorzugten Weiterbildung dieser Ausführungsform sind an der Verpackung am Übergangsbereich eine innenliegenden Gummierung und/oder innenliegende Dichtwülste und/oder eine Versteifung, insbesondere eine U-förmige Versteifung, ausgebildet. Durch die Gummierung (Gummibeschichtung) wird die Dichtwirkung aufeinanderliegender Innenseiten der Verpackung verbessert. Aneinanderliegende Dichtwülste (in der Regel auch aus Gummi) erreichen ebenfalls eine verbesserte Flüssigkeitsabdichtung, insbesondere wenn sie so angeordnet sind, dass sie im abgeknickten Zustand ineinander greifen. Mittels einer Versteifung ist es möglich, aufeinander liegende Wände der Verpackung über die Versteifung zu verspannen (insbesondere mit Fixierungsmitteln), und so einen dichten Kontakt zwischen den Innenseiten der Wände sicherzustellen.

Besonders bevorzugt ist auch eine Weiterbildung, bei der ein Fixierungsmittel, mit dem die Verpackung im abgeknickten Zustand fixiert werden kann, vorgesehen ist, insbesondere wobei das Fixierungsmittel einen oder mehrere Klebepunkte oder Klebestreifen umfasst. Bei dieser Weiterbildung braucht der abgeknickte Zustand nicht manuell über die gesamte Aktivierungszeit gesichert werden. Die Fixiermittel können auch dazu eingesetzt werden, die Wände der Verpackung zu spannen, um die Dichtwirkung im abgeknickten Zustand zu verbessern, etwa über eine Kante einer Versteifung.

Bei einer anderen, vorteilhaften Ausführungsform ist ein Verschlussmittel vorgesehen, mit dem der Übergangsbereich verschließbar ist, insbesondere wobei das Verschlussmittel, beispielsweise ein Stopfen, innerhalb der Verpackung angeordnet ist und von außen durch die Verpackung hindurch betätigbar ist.

Mittels eines Verschlussmittels, gegebenenfalls ergänzt durch ein Gegenlager, kann eine sehr gute Abdichtung erzielt werden. Ein erfindungsgemäßes äußeres, separates Verschlussmittel wäre zum Beispiel eine Klemme, die den Übergangsbereich zusammendrückt. Bevorzugt sind jedoch integrierte und/oder unverlierbare Verschlussmittel.

Eine vorteilhafte Ausführungsform sieht vor, dass der Übergangsbereich ein Ventil umfasst, welches einen Rückfluss von Benetzungsflüssigkeit vom Benetzungsbereich in den Vorratsbereich sperrt. Besonders bevorzugt ist ein Flatterventil. Mit einem Ventil erfolgt die Abdichtung des Benetzungsbereichs automatisch ohne manuelles Zutun des Verwenders des Kathetersystems. Ein sehr enger Durchlass, d.h. ein Übergangsbereich mit sehr engem Durchflussquerschnitt (etwa 1 mm² oder weniger), welcher nur unter Ausübung von äußerem Druck einen nennenswerten Durchfluss von Benetzungsflüssigkeit ermöglicht, kann die Funktion eines Ventils erfindungsgemäß erfüllen. In diesem Fall muss das Depot bzw. der Vorratsbereich mit Kraft in den Benetzungsbereich ausgequetscht werden, und bei einfachem Verschwenken der Verpackung im Rahmen von Schritt d) oder e) des erfindungemäßen Verfahrens kommt es zu keinem merklichen Rückfluss.

Bei einer bevorzugten Ausführungsform ist der Katheter vollständig im Benetzungsbereich angeordnet. In diesem Fall braucht kein Durchgang zu einem Nachbarraum, in dem ein übriger (unbeschichteter) Teil des Katheters angeordnet ist, abgedichtet zu werden. Der Verpackungsaufbau ist einfach und kostengünstig.

Eine andere, vorteilhafte Ausführungsform des erfindungsgemäßen Kathetersystems sieht vor, dass im Vorratsbereich eine separate Flüssigkeitspackung als Depot angeordnet ist. Die Flüssigkeitspackung kann bei der Produktion einfach zwischen Oberseite und Unterseite der Verpackung eingelegt werden. Die Flüssigkeitspackung ist beispielsweise eine Alupackung.

Bei einer alternativen Ausführungsform ist der Vorratsbereich selbst als Depot für die Benetzungsflüssigkeit ausgebildet. Dadurch kann Material eingespart werden.

Besonders bevorzugt ist eine Ausführungsform, bei der der Übergangsbereich an einem Endbereich des länglichen Benetzungsbereichs mündet. Dies vereinfacht das Einleiten der Benetzungsflüssigkeit in den Benetzungsbereich, wobei der Vorratsbereich nach oben und der Benetzungsbereich nach unten gehalten wird.

Eine bevorzugte Weiterbildung dieser Ausführungsform sieht vor, dass in dem Endbereich, an dem der Übergangsbereich mündet, die Einführspitze des Katheters angeordnet ist. Diese Geometrie ermöglicht ein besonders intensives Tränken der Einführspitze bei und nach Schritt d) des erfindungsgemäßen Verfahrens. Weiterhin bietet sich der Übergangsbereich zum Aufreißen der Verpackung und Entnehmen des Katheters an, da der Vorratsbereich seine Funktion zu diesem Zeitpunkt bereits erfüllt hat. Die zuerst einzuführende Einführspitze sollte zuerst aus der Verpackung entnommen werden können, um eine Kontaminationsgefahr im hinteren Katheterteil zu vermindern (Das Einführen des Katheters in die Harnröhre und das allmähliche Herausführen des Katheters aus der Verpackung finden zur Kontaminationsvermeidung oftmals gleichzeitig statt, wobei der Katheter noch teilweise mittels der Verpackung ergriffen werden kann).

Bevorzugt ist auch eine Ausführungsform, bei der die Verpackung im Übergangsbereich eine Sollschwachstelle für das öffnen der Verpackung und die Entnahme des Katheters aufweist. Der Übergangsbereich bietet sich als Sollschwachstelle an, da der Vorratsbereich seine Funktion bereits erfüllt hat und daher problemlos abgetrennt werden kann.

Bevorzugt ist auch eine Ausführungsform, bei der auf den Katheter im Bereich der Einführspitze eine Einführhilfe aufgesetzt ist oder eine Einführhilfe in der Verpackung in der Nähe der Einführspitze gelagert ist. Mit der Einführhilfe kann eine direkte Berührung (und möglicherweise Kontamination) des Katheters vor dem Einführen in die Harnröhre vermieden werden

Schließlich fällt noch in den Rahmen der vorliegenden Erfindung eine Verwendung eines oben beschriebenen, erfindungsgemäßen Kathetersystems in einem oben beschriebenen, erfindungsgemäßen Benetzungsverfahren.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

Die Erfindung ist in der Zeichnung dargestellt und wird anhand von Ausführungsbeispielen näher erläutert. Es zeigt:
- Fig. 1: eine schematische Aufsicht auf ein erfindungsgemäßes Kathetersystem zur Durchführung des erfindungsgemäßen Benetzungsverfahrens;
- Fig. 2a: eine schematische seitliche Ansicht auf das Kathetersystem von Fig. 1, vor Schritt a) des erfindungsgemäßen Verfahrens;
- Fig. 2b: das Kathetersystem von Fig. 2a, nach Schritt b) des erfindungsgemäßen Verfahrens;
- Fig. 2c: das Kathetersystem von Fig. 2a, nach Schritt c) des erfindungsgemäßen Verfahrens;
- Fig. 2d: das Kathetersystem von Fig. 2a, nach Schritt d) des erfindungsgemäßen Verfahrens;
- Fig. 3a: eine schematische Aufsicht auf eine weitere Ausführungsform eines erfindungsgemäßen Kathetersystems mit einer U-förmigen Aussteifung an einer Biegelinie, im Detailausschnitt am Übergangsbereich;
- Fig. 3b: das Kathetersystem von Fig. 3a, im schematischen seitlichen Querschnitt vor einer Abknickung;
- Fig. 3c: das Kathetersystem von Fig. 3b nach einer Abknickung zur Abdichtung des Übergangsbereichs;
- Fig. 4a: ein schematischer seitlicher Querschnitt einer weiteren Ausführungsform eines erfindungsgemäßen Kathetersystems mit drei Dichtwülsten, im Detailausschnitt am Übergangsbereich, vor einer Abknickung;
- Fig. 4b: das Kathetersystem von Fig. 4a, nach einer Abknickung zur Abdichtung des Übergangsbereichs;
- Fig. 5: eine schematische Aufsicht auf eine weitere Ausführungsform eines erfindungsgemäßen Kathetersystems mit einem Stopfen, im Detailausschnitt am Übergangsbereich;
- Fig. 6: eine schematische Aufsicht auf das Kathetersystem von Fig. 1, nach Abtrennen des Vorratsbereich und nach teilweisem Herausschieben des Katheters;4
- Fig. 7: eine schematische Aufsicht auf eine weitere Ausführungsform eines erfindungsgemäßen Kathetersystems, mit einer in der Verpackung angeordneten Einführhilfe.

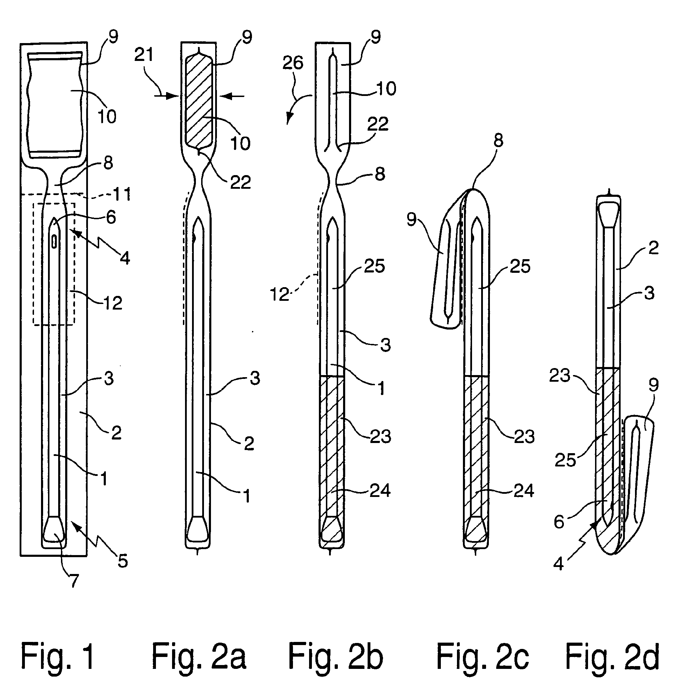

**Figur 1** zeigt in schematischer Aufsicht eine erste Ausführungsform eines erfindungsgemäßen Kathetersystems. Das Kathetersystem umfasst einen hydrophil beschichteten, aus Kunststoff gefertigten, flexiblen Katheter 1 und eine Verpackung 2, in der der Katheter 1 angeordnet ist. Die Verpackung 2 wird hier im Wesentlichen durch zwei aufeinander geklebte oder verschweißte, transparente Kunststofffolien ausgebildet, so dass ein Verwender (Patient) in das Innere der Verpackung sehen kann. Zwischen den Kunststofffolien sind diverse Hohlräume ausgebildet, die der Verpackung 2 ihre Funktion verleihen.

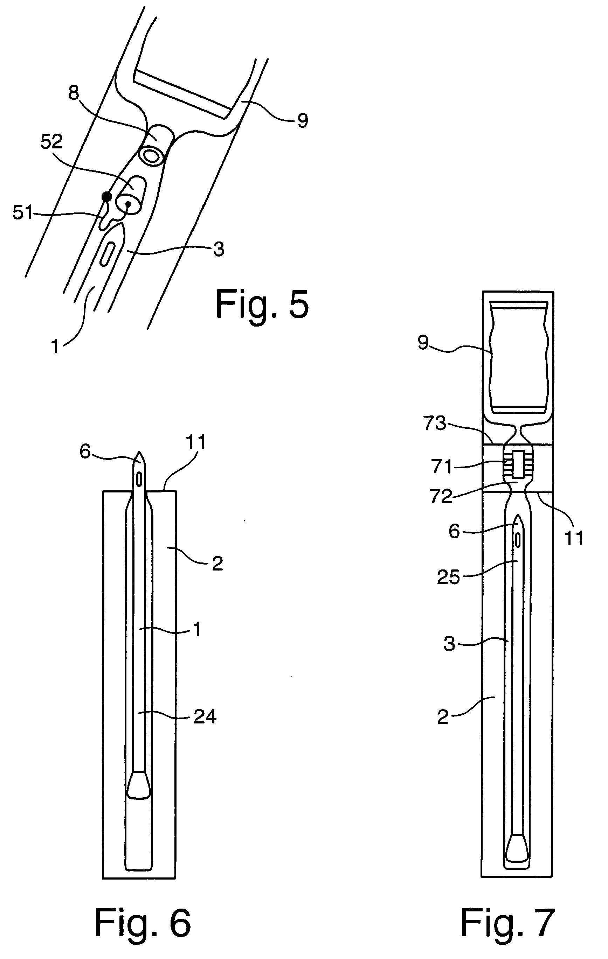

Der Katheter 1 ist in einem länglichen Benetzungsbereich 3 angeordnet, der sich von einem in Fig. 1 oberen, ersten Endbereich oder Ende 4 zu einem zweiten, in Fig. 5 unteren Endbereich oder Ende 5 erstreckt. Der Katheter 1 ist so orientiert, dass sein vorderes Einführende 6 im ersten Endbereich 4 angeordnet ist, und sein hinteres Trichterelement 7 im zweiten Endbereich 5 angeordnet ist. Der Benetzungsbereich 3 umschließt dabei den Katheter 1 so eng, dass dieser gerade noch samt dem Trichterelement 7 (welches den größten Durchmesser am Katheter 1 ausbildet) durch den Benetzungsbereich 3 durchgeführt (geschoben) werden kann.

In den ersten Endbereich 4 des länglichen Benetzungsbereichs 3 mündet ein Übergangsbereich 8. Der Übergangsbereich 8 verbindet den Benetzungsbereich 3 mit einem Vorratsbereich 9 der Verpackung 2. In dem Vorratsbereich 9 ist ein separates Depot 10 angeordnet, welches mit einer Benetzungsflüssigkeit, hier sterile wässrige 0,9%ige NaCl-Lösung (physiologische Kochsalzlösung), gefüllt ist. Der Übergangsbereich 8 ist sowohl gegenüber dem Vorratsbereich 9 als auch gegenüber dem Benetzungsbereich 3 verengt ausgebildet.

An der Verpackung 2 ist eine Biegelinie 11 am Übergangsbereich 8 markiert. An der Biegelinie 11 ist die Verpackung 2 auch etwas schwächer ausgebildet, so dass diese gleichzeitig eine Sollschwachstelle geeignet zum Aufreißen der Verpackung 2 darstellt.

Auf der Außenseite der Verpackung 2 ist außerdem ein Klebesteifen 12 (gestrichelt markiert) vorgesehen, beispielsweise ausgebildet durch ein zweiseitig klebendes Band.

Der Benetzungsbereich 3 hat ein Volumen von Vbb (in der gezeigten Ausführungsform mit einer Verpackung 2 aus flexiblen Kunststofffolien bezeichnet Vbb den maximalen Volumeninhalt des Benetzungsbereichs 3). Der Katheter 1, der in der gezeigten Ausführungsform vollständig im Benetzungsbereich 3 angeordnet ist, nimmt ein Volumen (Verdrängungsvolumen) von Vkat ein, so dass im Benetzungsbereich 3 ein freies Volumen Vfrei=Vbb-Vkat verbleibt. Das Depot 10 beinhaltet ein Volumen Vfl an Benetzungsflüssigkeit.

In den Figuren 2a bis 2d wird die erfindungsgemäße Benetzung des Katheters 1 in der Verpackung 2 illustriert. Die Figuren 2a-2d zeigen dabei jeweils seitliche Querschnittsansichten.

Zunächst wird die Verpackung 2 so gehalten, dass der Vorratsbereich 9 oben und der Benetzungsbereich 3 unten ist; im einfachsten Fall wird die Verpackung 2 dazu senkrecht gehalten, siehe **Fig. 2a****.** Sodann wird das Depot 10 geöffnet, hier durch Ausübung von seitlichem Druck (vgl. Pfeile 21), so dass das Depot 10 am unteren Rand 22 aufplatzt.

Sodann ergießt sich die im Depot 10 enthaltene Benetzungsflüssigkeit der Schwerkraft folgend vom Vorratsbereich 9 nach unten durch den Übergangsbereich 8 in den Benetzungsbereich 3. Dadurch wird der Benetzungsbereich 3 in einem unteren Teil mit der Benetzungsflüssigkeit 23 (schraffiert gezeichnet) aufgefüllt (überflutet). Der Katheter 1 steht mit seinem hinteren Teil 24 in der Benetzungsflüssigkeit 23 (vgl. **Fig. 2b****),** wodurch die hydrophile Beschichtung des Katheters 1 am hinteren Teil 24 zuverlässig aktiviert wird. Das entleerte Depot 10 verbleibt im Vorratsbereich 9. Im gezeigten Ausführungsbeispiel wird der Benetzungsbereich 3 etwa zur Hälfte mit Benetzungsflüssigkeit 23 gefüllt. Mit anderen Worten, das Volumen Vfl der Benetzungsflüssigkeit 23 entspricht etwa dem halben freien Volumen Vfrei des Benetzungsbereichs 3. Der vordere Teil 25 des Katheters 1 kommt bei der Entleerung des Depots 10 nur kurz, und stellenweise womöglich gar nicht in Kontakt mit der Benetzungsflüssigkeit.

Zur Vorbereitung der Aktivierung der hydrophilen Beschichtung des Katheters 1 im vorderen Teil 25 wird nun der Übergangsbereich 8 durch Umknicken der Verpackung 2 an der Biegelinie 11 abgedichtet. Dabei wird der Vorratsbereich 9 in Fig. 2b nach links (vgl. Pfeil 26 in Fig. 2b) auf den Klebestreifen 12 (gestrichelt gezeichnet) gefaltet.

Der Klebestreifen 12 fixiert die gefaltete Lage des Vorratsbereichs 9 (vgl. **Fig.** 2c). Infolge des Knicks an der Biegelinie 11 wird der Durchflussquerschnitt für Benetzungsflüssigkeit 23 am Obergangsbereich 8 stark verringert, so dass ein Rückfluss von Benetzungsflüssigkeit 23 in den Vorratsbereich 9 im Wesentlichen blockiert ist.

Anschließend wird die Verpackung 2 verschwenkt, so dass der Übergangsbereich 8 unterhalb des Benetzungsbereichs 3 angeordnet ist; in **Fig. 2d** wurde dazu die Verpackung 2 um 180° gedreht, so dass der Katheter 1 wieder senkrecht steht, mit deinem Einführende 6 nach unten gerichtet. Infolge der Verschwenkung ist die Benetzungsflüssigkeit 23 in den ersten Endbereich 4 des Benetzungsbereichs 3 geflossen. Nun steht der vordere Teil 25 des Katheters 1 in der Benetzungsflüssigkeit 23, wodurch auch die hydrophile Beschichtung des Katheters 1 am vorderen Teil 25 zuverlässig benetzt wird. Der bereits intensiv getränkte hintere Teil 24 des Katheters 1 ragt aus der Benetzungsflüssigkeit 23 heraus. Aufgrund der Abdichtung des Obergangsbereich 8 kommt es zu keinem wesentlichen Fluss von Benetzungsflüssigkeit 23 in den Vorratsbereich 9 in dieser Lage der Verpackung 2.

Nachdem alle Abschnitte des Katheters 1 einmal in der Benetzungsflüssigkeit 23 standen, ist der Katheter 1 vollständig intensiv getränkt worden, und nach ausreichender Wartezeit (ca. 15-30 Sekunden) kann der Katheter 1 ausgepackt und in die Harnröhre eines Patienten eingeführt werden.

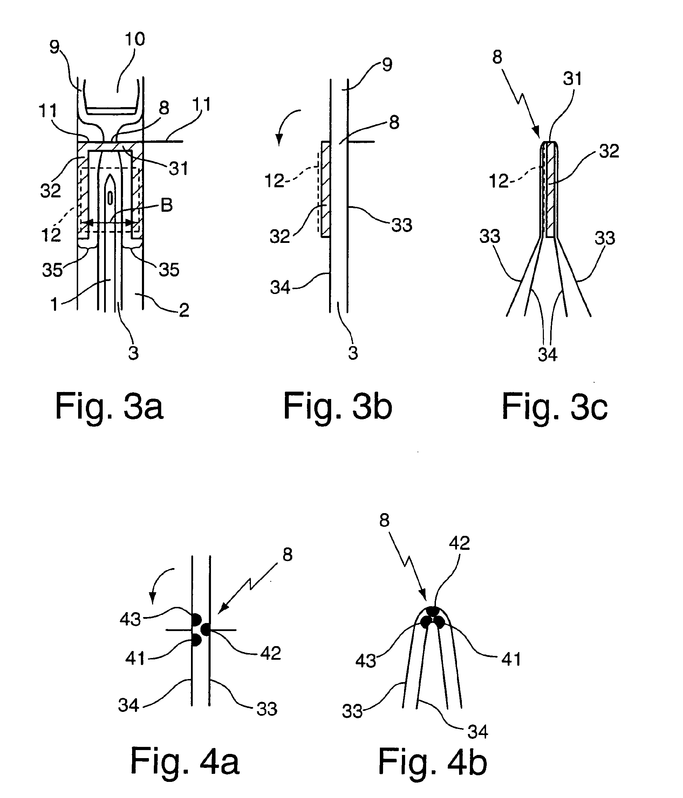

In den Figuren 3a-3c wird die Wirkungsweise einer U-förmigen Versteifung illustriert, die die Abdichtung an einer Biegelinie verbessern kann.

In der schematischen Aufsicht von **Fig. 3a** ist ein Ausschnitt aus einem erfindungsgemäßen Kathetersystem am Übergangsbereich 8 illustriert. Die Basis 31 einer U-förmigen Versteifung 32, die an der Verpackung 2 ausgebildet (z. B. aufgeklebt) ist, verläuft parallel zu einer Biegekante 11. Wird nun der Vorratsbereich 9 wie im seitlichen Querschnitt von **Fig. 3b** mit einem Pfeil markiert auf den Benetzungsbereich 3 bzw. den dort befestigten Klebestreifen 12 heruntergeklappt, so kann die Kunststofffolie der Verpackung 2, insbesondere deren äußere Wand 33, über die obere Kante der Basis 31 der U-förmigen Versteifung 32 gespannt werden (**Fig.** 3c).

Eine besonders gute Spannung wird erreicht, wenn die Breite B des Klebestreifens 12 so groß ist, dass die beiden Schenkel der U-förmigen Versteifung 32 mit überspannt werden, und auch ein jeweils Bereich 35 der Verpackung 2 überspannt wird, an dem die äußere Wand 33 und die innere Wand 34 der Verpackung 2 aneinander befestigt sind (etwa verschweißt sind), vgl. Fig. 3a.

Eine andere Verbesserungsmöglichkeit der Dichtwirkung beim Abknicken ist in den schematischen seitlichen Querschnitten von **Fig. 4a, 4b** dargestellt. An den Innenseiten der Wände 33, 34 der Verpackung 2 sind im Übergangsbereich 8 insgesamt drei Dichtwülste 41, 42, 43 ausgebildet, wobei im abgeknickten Zustand der Dichtwulst 42 der Außenwand 33 zwischen die gegenüberliegenden Dichtwülste 41, 43 der Innenwand 34 eingreift und an den Dichtwülsten 41, 42 anliegt.

In der **Figur 5** ist eine andere Möglichkeit der Abdichtung eines Übergangsbereichs 8 bei einer anderen Ausführungsform eines erfindungsgemäßen Kathetersystems dargestellt. Der Übergangsbereich 8 ist hier als eine starrer Rohrabschnitt ausgebildet. Im Benetzungsbereich 3 ist an einer Leine 51 ein Stopfen 52 befestigt, der durch die Verpackung 2 manuell in den Rohrabschnitt eingeführt werden kann und diesen dann verschließt.

**Figur 6** erläutert die Entnahme eines Katheters aus einem erfindungsgemäßen Kathetersystem wie in Fig. 1 dargestellt. Die Verpackung 2 wurde an der Biegelinie 11 aufgerissen, wodurch der Benetzungsbereich 3 geöffnet wurde. Der Katheter 1 kann dann mit der Einführspitze 6 voraus aus der Verpackung 2 entnommen werden, wobei die Verpackung 2 ein direktes Berühren des Katheters 1 im hinteren Teil 24 vermeiden hilft.

Um den Katheter 1 auch an einem vorderen Teil 25 ohne direkte Berührung ergreifen zu können, ist in der Ausführungsform eines Kathetersystems gemäß der schematischen Aufsicht von **Fig. 7** eine Einführhilfe 71 in der Verpackung 2 vorgesehen. Die Einführhilfe 71 ist in einem Lagerraum 72 zwischen Depot 10 und Katheter 1 angeordnet. Die Verpackung 2 weist eine Biegelinie 11 und eine Sollschwachstelle 73 zum Aufreißen der Verpackung 2 auf. Alternativ kann auch an der Sollschwachstelle 73 eine Abknickung erfolgen. Zum Entnehmen des Katheters 1 wird dieser zunächst durch eine Bohrung der Einführhilfe 71 geschoben und sodann die Verpackung 2 an Sollschwachstelle 73 aufgerissen. Der Katheter 1 kann dann zusammen mit der aufgesetzten Einführhilfe 71 herausgeschoben werden, und die Einführspitze 6 kann beim Einführen in die Harnröhre mit der Einführhilfe 71 leicht ausgerichtet werden. Eine direkte Berührung des Katheters 1 nahe der Einführspitze 6 kann damit vermieden werden; stattdessen genügt es, die Einführhilfe 71 von außen zu berühren. Die Einführhilfe 71 wird nicht in die Harnröhre eingeführt.

Zusammenfassend beschreibt die vorliegende Erfindung ein Verfahren zum Benetzen eines hydrophilen Katheters innerhalb einer geschlossenen Verpackung mit einer reduzierten Menge an Benetzungsflüssigkeit. Ein Benetzungsraum der Verpackung wird soweit mit Benetzungsflüssigkeit gefüllt, dass nur ein Teil der hydrophilen Beschichtung eines dortigen Katheters überflutet wird. Sodann wird der Benetzungsraum abgedichtet, und die Verpackung so verschwenkt, dass auch die übrigen Teile der hydrophilen Beschichtung des Katheters überflutet oder zumindest intensiv umspült werden. Durch die Abdichtung ist Abfluss von Benetzungsflüssigkeit aus dem Benetzungsraum ausgeschlossen. Die Abdichtung erfolgt bevorzugt ohne von der Verpackung separate Hilfsmittel, beispielsweise nur durch manuelles Umknicken. Abdichtungsort ist insbesondere die Verbindungsstelle zwischen Benetzungsbereich und Vorratsbereich für die Benetzungsflüssigkeit.

## Patentansprüche

1. Verfahren zur Benetzung eines hydrophilen Urinal-Katheters (1) innerhalb einer geschlossenen Verpackung (2) des Katheters (1) wobei die Verpackung (2) aufweist
- einen länglichen Benetzungsbereich (3), der zumindest einen zu benetzenden Teil des hydrophilen Urinal-Katheters (1) enthält, und
- einen Vorratsbereich (9) mit einem Depot (10) für eine Benetzungsflüssigkeit (23),
wobei das Depot (10) geöffnet werden kann, so dass die Benetzungsflüssigkeit (23) vom Vorratsbereich (9) durch einen Übergangsbereich (8) in den Benetzungsbereich (3) fließen kann, wobei das Verfahren folgende Schritte umfasst:
a) das Depot (10) wird geöffnet, insbesondere durch Ausübung eines äußeren Drucks auf das Depot (10) durch die Verpackung (2);
b) die Benetzungsflüssigkeit (23) fließt aus dem Vorratsbereich (9) in ein freies Volumen Vfrei des länglichen Benetzungsbereichs (3) und füllt dieses zu maximal 2/3 mit Benetzungsflüssigkeit (23), insbesondere fließt die Benetzungsflüssigkeit (23) durch Schwerkraft zu einem Ende (5) des länglichen Benetzungsbereichs (3), das vom Übergangsbereich (8) abgewandt ist, wobei das freie Volumen Vfrei das Volumen Vbb des Benetzungsbereichs (3) abzüglich des Volumens Vkat des im Benetzungsbereichs (3) angeordneten Teils des Katheters (1) ist;
c) anschließend wird der Übergangsbereich (8) abgedichtet, so dass ein Rückfluss von Benetzungsflüssigkeit (23) aus dem Benetzungsbereich (3) in den Vorratsbereich (9) blockiert ist;
d) die Verpackung (2) wird geschwenkt, insbesondere so dass die Benetzungsflüssigkeit (23) durch Schwerkraft zu einem Ende (4) des länglichen Benetzungsbereichs (3) fließt, welches dem Übergangsbereich (8) nahegelegen ist.

2. Verfahren nach Anspruch 1, **dadurch gekennzeichnet, dass** nach Schritt
d) wenigstens 15 Sekunden, bevorzugt wenigstens 30 Sekunden, vergehen, bevor der Katheter (1) ausgepackt wird und in eine Harnröhre eines Patienten einführbar ist.

3. Verfahren nach einem der vorhergehenden Ansprüche, **dadurch gekennzeichnet, dass** nach Schritt d) ein weiterer Schritt e) erfolgt, mit e) Die Verpackung (2) wird hin- und hergeschwenkt, so dass die Benetzungsflüssigkeit (23) im länglichen Benetzungsbereich (3) zwischen dessen Enden (4, 5) hin- und herfließt.

4. Verfahren nach einem der vorhergehenden Ansprüche, **dadurch gekennzeichnet, dass** in Schritt c) die Verpackung (2) zum Abdichten am Übergangsbereich (8) umgeknickt wird.

5. Verfahren nach einem der vorhergehenden Ansprüche, **dadurch gekennzeichnet, dass** in Schritt b) das freie Volumen Vfrei maximal zu 1/2 gefüllt wird

6. Kathetersystem, umfassend
- einen Urinal-Katheter (1) mit einer hydrophilen Beschichtung und
- eine Verpackung (2), in der der Katheter (1) angeordnet ist, wobei die Verpackung (2) aufweist
- einen länglichen Benetzungsbereich (3), der zumindest einen zu benetzenden Teil des Katheters (1) enthält, und
- einen Vorratsbereich (9) mit einem Depot (10) für eine Benetzungsflüssigkeit (23), wobei das Depot (10) geöffnet werden kann, so dass die Benetzungsflüssigkeit (23) vom Vorratsbereich (9) durch einen Übergangsbereich (8) in den Benetzungsbereich (3) fließt, **dadurch gekennzeichnet, dass** der Übergangsbereich (8) von einem ersten geöffneten Zustand in einen zweiten abgedichteten Zustand überführbar ist, sich der Übergangsbereich (8) beim Öffnen des Depots (10) im geöffneten Zustand befindet und nach Abfluss der Benetzungsflüssigkeit (23) in den Benetzungsbereich (3) in den abgedichteten Zustand gebracht ist, so dass ein Rückfluss von Benetzungsflüssigkeit (23) aus dem Benetzungsbereich (3) in den Vorratsbereich (9) verhindert ist, wobei der abgedichtete Zustand durch ein Abknicken im Übergangsbereich (8) zwischen Vorratsbereich (9) und Benetzungsbereich (3) erzeugt ist und für das Volumen Vfl der Benetzungsflüssigkeit (23) des Depots (10), das Volumen Vbb des Benetzungsbereichs (3) und das Volumen Vkat des Teils des Katheters (1), welcher im Benetzungsbereich (3) angeordnet ist, gilt: Vfl ≤ 2/3*(Vbb-Vkat), und bevorzugt Vfl ≤ 1/2*(Vbb-Vkat).

7. Kathetersystem nach Anspruch 6, **dadurch gekennzeichnet, dass** an der Verpackung (2) am Übergangsbereich (8) eine innenliegenden Gummierung und/oder innenliegende Dichtwulste (41, 42, 43) und/oder eine Versteifung, insbesondere eine U-förmige Versteifung (32), ausgebildet sind.

8. Kathetersystem nach Anspruch 6 oder 7, **dadurch gekennzeichnet, dass** ein Fixierungsmittel, mit dem die Verpackung (2) im abgeknickten Zustand fixiert werden kann, vorgesehen ist, insbesondere wobei das Fixierungsmittel einen oder mehrere Klebepunkte oder Klebestreifen (12) umfasst.

9. Kathetersystem nach Anspruch 6, **dadurch gekennzeichnet, dass** ein Verschlussmittel vorgesehen ist, mit dem der Übergangsbereich (8) verschließbar ist, insbesondere wobei das Verschlussmittel, beispielsweise ein Stopfen (52), innerhalb der Verpackung (2) angeordnet ist und von außen durch die Verpackung (2) hindurch betätigbar ist.

10. Kathetersystem nach Anspruch 6, **dadurch gekennzeichnet, dass** der Übergangsbereich (8) ein Ventil umfasst, welches einen Rückfluss von Benetzungsflüssigkeit (23) vom Benetzungsbereich (3) in den Vorratsbereich (9) sperrt.

11. Kathetersystem nach einem der Ansprüche 6 bis 10, **dadurch gekennzeichnet, dass** der Katheter (1) vollständig im Benetzungsbereich (3) angeordnet ist.

12. Kathetersystem nach einem der Ansprüche 6 bis 11, dass im Vorratsbereich (9) eine separate Flüssigkeitspackung (10) als Depot angeordnet ist.

13. Kathetersystem nach einem der Ansprüche 6 bis 12, **dadurch gekennzeichnet, dass** der Vorratsbereich (9) selbst als Depot für die Benetzungsflüssigkeit (23) ausgebildet ist.

14. Kathetersystem nach einem der Ansprüche 6 bis 13, **dadurch gekennzeichnet, dass** der Übergangsbereich (8) an einem Endbereich (4, 5) des länglichen Benetzungsbereichs (3) mündet.

15. Kathetersystem nach Anspruch 14, **dadurch gekennzeichnet, dass** in dem Endbereich (4), an dem der Übergangsbereich (8) mündet, die Einführspitze (6) des Katheters (1) angeordnet ist.

16. Kathetersystem nach einem der Ansprüche 6 bis 15, **dadurch gekennzeichnet, dass** die Verpackung (2) im Übergangsbereich (8) eine Sollschwachstelle (11; 73) für das Öffnen der Verpackung (2) und die Entnahme des Katheters (1) aufweist.

17. Kathetersystem nach einem der Ansprüche 6 bis 16, **dadurch gekennzeichnet, dass** auf den Katheter (1) im Bereich der Einführspitze (6) eine Einführhilfe aufgesetzt ist oder eine Einführhilfe (71) in der Verpackung (2) in der Nähe der Einführspitze (6) gelagert ist.

18. Verwendung eines Kathetersystems nach einem der Ansprüche 6 bis 17 in einem Verfahren nach einem der Ansprüche 1 bis 4.

## Claims

1. The method for wetting a hydrophilic urine catheter (1) within a closed package (2) of the catheter (1), said package (2) comprising
- an elongate wetting region (3) containing at least a hydrophilic urine catheter part which is to be wetted, and
- a storage area (9) with a depot (10) for a wetting fluid (23),
said depot (10) being openable so that the wetting fluid (23) can flow from the storage area (9) through a transition area (8) into the wetting region (3), wherein said method comprises the following steps:
a) the depot (10) is opened, especially by applying external pressure to the depot (10) through the package (2);
b) the wetting fluid (23) flows from the storage area (9) into a free volume Vfree of the elongate wetting region (3) and fills said free volume up to a maximum of two thirds, in particular the wetting fluid (23) flows, due to the force of gravity, to an end (5) of the elongate wetting region (3) that faces away from the transition area (8), said free volume Vfree being the volume Vwr of the wetting region (3) minus the volume Vcat of the catheter part arranged in the wetting region (3);
c) the transition area (8) is then sealed so that a return flow of wetting fluid (23) from the wetting region (3) into the storage area (9) is blocked;
d) the package (2) is turned or swiveled, in particular such that, due to the force of gravity, the wetting fluid (23) will flow to an end (4) of the elongate wetting region (3) which is located close to the transition area (8).

2. The method according to claim 1, **characterized in that**, after step d), at least 15 seconds, preferably at least 30 seconds, elapse before the catheter (1) is unwrapped and introducable in a patient's urethra.

3. The method according to one of the preceding claims, **characterized in that**, after step d), a further step e) is executed, where: e) the package (2) is swiveled back and forth so that the wetting fluid (23) will flow back and forth in the elongate wetting region (3) between the ends (4, 5) of the latter.

4. The method according to one of the preceding claims, **characterized in that** in step c) the package (2) is kinked at the transition area (8) for the purpose of sealing.

5. The method according to one of the preceding claims, **characterized in that** in step b) the free volume Vfree is filled up to a maximum of 1/2.

6. The catheter system comprising
- a urine catheter (1) with a hydrophilic coating and
- a package (2) in which said catheter (1) is arranged, said package (2) comprising
- an elongate wetting region (3) containing at least a catheter part which is to be wetted, and
- a storage area (9) with a depot (10) for a wetting fluid (23), said depot (10) being openable so that the wetting fluid (23) flows from the storage area (9) through a transition area (8) into the wetting region (3), **characterized in that** the transition area (8) can be changed from a first, open condition to a second, sealed condition, that the transition area (8) is in the open condition when the depot (10) is being opened and is in the sealed condition when the wetting fluid (23) has been discharged into the wetting region (3), so that a return flow of wetting fluid (23) from the wetting region (3) into the storage area (9) is prevented, the sealed condition being established by kinking in the transition area (8) between the storage area (9) and the wetting region (3), and that for the volume Vfl of the wetting fluid (23) of the depot (10), the volume Vwr of the wetting region (3) and the volume Vcat of the catheter part arranged in said wetting region (3), the following holds true: Vfl ≤ 2/3 * (Vwr-Vcat), and preferably Vfl ≤ 1/2 * (Vwr-Vct).

7. The catheter system according to claim 6, **characterized in that**, in the transition area (8), the package (2) has formed thereon an interior rubber coating and/or interior sealing beads (41, 42, 43) and/or a stiffener, in particular a U-shaped stiffener (32).

8. The catheter system according to claim 6 or 7, **characterized in that** fixing means are provided, with the aid of which the package (2) can be fixed in its kinked condition, said fixing means comprising especially one or a plurality of adhesive spots or adhesive tapes (12).

9. The catheter system according to claim 6, **characterized in that** closure means are provided, with aid of which the transition area (8) can be closed, said closure means, e.g. a plug (52), being especially arranged in the interior of the package (2) and being operable from outside through the package (2).

10. The catheter system according to claim 6, **characterized in that** the transition area (8) comprises a valve, which blocks a return flow of wetting fluid (23) from the wetting region (3) into the storage area (9).

11. The catheter system according to one of the claims 6 to 10, **characterized in that** the catheter (1) is fully arranged within the wetting region (3).

12. The catheter system according to one of the claims 6 to 11, **characterized in that** the storage area (9) has provided therein a separate fluid package (10) as a depot.

13. The catheter system according to one of the claims 6 to 12, **characterized in that** the storage area (9) itself is configured as depot for the wetting fluid (23).

14. The catheter system according to one of the claims 6 to 13, **characterized in that** the transition area (8) opens into an end area (4, 5) of the elongate wetting region (3).

15. The catheter system according to claim 14, **characterized in that** the insertable tip (6) of the catheter (1) is arranged in the end area (4, 5) into which the transition area (8) opens.

16. The catheter system according to one of the claims 6 to 15, **characterized in that** the package (2) includes in the transition area (8) a predetermined weak point (11; 73) for opening the package (2) and removing the catheter (1).

17. The catheter system according to one of the claims 6 to 16, **characterized in that**, in the area of the insertable tip (6), the catheter (1) has attached thereto an insertion aid, or that an insertion aid (71) is supported in the package (2) in the vicinity of the insertable tip (6).

18. The use of a catheter system according to one of the claims 6 to 17 in a method according to one of the claims 1 to 4.

## Revendications

1. Procédé pour assurer un mouillage d'un cathéter urinaire (1) hydrophile à l'intérieur d'un emballage (2) fermé du cathéter (1), l'emballage (2) comprenant
- une zone de mouillage (3) allongée, qui renferme au moins une partie à mouiller du cathéter urinaire (1) hydrophile, et
- une zone de réserve (9) avec une chambre de stockage (10) pour un liquide de mouillage (23), procédé d'après lequel
la chambre de stockage (10) peut être ouverte de manière à ce que le liquide de mouillage (23) puisse s'écouler de la zone de réserve (9), à travers une zone de transition (8), dans la zone de mouillage (3), le procédé comprenant les étapes suivantes :
a) ouverture de la chambre de stockage (10), notamment en exerçant une pression extérieure sur la chambre de stockage (10) à travers l'emballage (2) ;
b) écoulement du liquide de mouillage (23) à partir de la zone de réserve (9) jusque dans un volume libre Vfrei de la zone de mouillage (3) allongée, et remplissage de celle-ci au maximum à 2/3 avec du liquide de mouillage (23), et notamment écoulement du liquide de mouillage (23) par gravité vers une extrémité (5) de la zone de mouillage (3) allongée, qui est à l'opposé de la zone de transition (8), le volume libre Vfrei étant égal au volume Vbb de la zone de mouillage (3) diminué du volume Vkat de la partie du cathéter (1) agencée dans la zone de mouillage (3) ;
c) ensuite fermeture étanche de la zone de transition (8) de manière à bloquer un écoulement de retour du liquide de mouillage (23) de la zone de mouillage (3) dans la zone de réserve (9) ;
d) pivotement de l'emballage (2), notamment de manière à ce que le liquide de mouillage (23) s'écoule par gravité vers une extrémité (4) de la zone de mouillage (3) allongée, qui est proche de la zone de transition (8).

2. Procédé selon la revendication 1, **caractérisé en ce qu'**après l'étape d), on attend au moins 15 secondes, de préférence au moins 30 secondes, avant de déballer le cathéter (1) et de l'introduire dans l'urètre d'un patient.

3. Procédé selon l'une des revendications précédentes, **caractérisé en ce qu'**après l'étape d), on effectue une autre étape e), l'étape e) consistant à faire pivoter en va-et-vient l'emballage (2), de manière à ce que le liquide de mouillage (23) s'écoule en va-et-vient dans la zone de mouillage (3) allongée, entre les extrémités (4, 5) de celle-ci.

4. Procédé selon l'une des revendications précédentes, **caractérisé en ce que** dans l'étape c), pour assurer la fermeture étanche, l'emballage (2) est replié sur lui-même dans la zone de transition (8).

5. Procédé selon l'une des revendications précédentes, **caractérisé en ce que** dans l'étape b), le volume libre Vfrei est rempli au maximum à ½.

6. Set de cathéter comprenant
- un cathéter urinaire (1) avec un revêtement hydrophile, et
- un emballage (2) dans lequel est agencé le cathéter (1), l'emballage (2) présentant
- une zone de mouillage (3) allongée, qui renferme au moins une partie à mouiller du cathéter (1), et
- une zone de réserve (9) avec une chambre de stockage (10) pour un liquide de mouillage (23), la chambre de stockage (10) pouvant être ouverte de manière à ce que le liquide de mouillage (23) s'écoule de la zone de réserve (9), en passant par une zone de transition (8), dans la zone de mouillage (3), **caractérisé en ce que** la zone de transition (8) peut être transférée d'un premier état ouvert à un deuxième état fermé de manière étanche, **en ce que** la zone de transfert (8) se trouve lors de l'ouverture de la chambre de stockage (10), dans l'état ouvert, et après écoulement du liquide de mouillage (23) dans la zone de mouillage (3), elle est amenée dans l'état fermé de manière étanche, de façon à empêcher ainsi un écoulement de retour du liquide de mouillage (23) de la zone de mouillage (3) à la zone de réserve (9), l'état de fermeture étanche étant produit en repliant sur elle-même la zone de transition (8) entre la zone de réserve (9) et la zone de mouillage (3), et **en ce que** pour le volume Vfl du liquide de mouillage (23) de la chambre de stockage (10), le volume Vbb de la zone de mouillage (3) et le volume Vkat de la partie du cathéter (1), qui est agencée dans la zone de mouillage (3), on a la relation suivante :
Vfl ≤ 2/3*(Vbb-Vkat), et de préférence Vfl ≤ 1/2*(Vbb-Vkat).

7. Set de cathéter selon la revendication 6, **caractérisé en ce que** sur l'emballage (2), au niveau de la zone de transition (8), sont formés un caoutchoutage situé à l'intérieur et/ou des bourrelets d'étanchéité (41, 42, 43) situés à l'intérieur et/ou un renfort de rigidification, notamment un renfort de rigidification en forme de U (32).

8. Set de cathéter selon la revendication 6 ou la revendication 7, **caractérisé en ce qu'**il est prévu un moyen de fixation à l'aide duquel l'emballage (2) peut être fixé dans l'état replié sur soi, le moyen de fixation comprenant notamment un ou plusieurs points adhésifs ou une bande adhésive (12).

9. Set de cathéter selon la revendication 6, **caractérisé en ce qu'**il est prévu un moyen de fermeture à l'aide duquel la zone de transition (8) peut être fermée, le moyen de fermeture, par exemple un bouchon (52), étant notamment agencé à l'intérieur de l'emballage (2), et peut être actionné à partir de l'extérieur, à travers l'emballage (2).

10. Set de cathéter selon la revendication 6, **caractérisé en ce que** la zone de transition (8) comprend une valve, qui bloque un écoulement de retour de liquide de mouillage (23) de la zone de mouillage (3) à la zone de réserve (9).

11. Set de cathéter selon l'une des revendications 6 à 10, **caractérisé en ce que** le cathéter (1) est agencé en totalité dans la zone de mouillage (3).

12. Set de cathéter selon l'une des revendications 6 à 11, **caractérisé en ce que** dans la zone de réserve (9) est agencé un emballage de liquide (10) séparé, en guise de chambre de stockage.

13. Set de cathéter selon l'une des revendications 6 à 12, **caractérisé en ce que** la zone de réserve (9) est réalisée elle-même en tant que chambre de stockage pour le liquide de mouillage (23).

14. Set de cathéter selon l'une des revendications 6 à 13, **caractérisé en ce que** la zone de transition (8) débouche au niveau d'une zone d'extrémité (4, 5) de la zone de mouillage (3) allongée.

15. Set de cathéter selon la revendication 14, **caractérisé en ce que** dans la zone d'extrémité (4) où débouche la zone de transition (8), est agencée la pointe d'introduction (6) du cathéter (1).

16. Set de cathéter selon l'une des revendications 6 à 15, **caractérisé en ce que** l'emballage (2) présente dans la zone de transition (8), une zone de moindre résistance programmée (11; 73) pour l'ouverture de l'emballage (2) et le prélèvement du cathéter (1).

17. Set de cathéter selon l'une des revendications 6 à 16, **caractérisé en ce que** sur le cathéter (1), dans la zone de la pointe d'introduction (6), est appliquée une aide à l'introduction, ou bien une aide à l'introduction (71) est stockée dans l'emballage (2) à proximité de la pointe d'introduction (6).

18. Utilisation d'un set de cathéter selon l'une des revendications 6 à 17 dans la mise en oeuvre d'un procédé selon l'une des revendications 1 à 4.
